# EUROPEAN PATENT SPECIFICATION

(11) **EP 0 335 814 B1**
(45) Date of publication and mention of the grant of the patent: **23.11.1994**
(21) Application number: 89600001.5
(22) Date of filing: 25.01.1989
(51) Int. Cl.: A61M 1/28

(54) **Peritoneal dialysis system**
Peritonealdialysesystem
Système de dialyse péritonéale

(30) Priority: 29.01.1988 GR 88010039
(43) Date of publication of application: 04.10.1989
(73) Proprietor: Anassis, Paul, Papagou, Attica (GR)
(72) Inventor: Anassis, Paul, Papagou, Attica (GR)
(74) Representative: Papastavrou, Kalliopi

(56) References cited:
- EP-A- 0 092 528

## Description

The invention refers to a tubes arrangement for the exchange of liquids (bring in and out) between the peritoneal cavity and two bags (one of the bags been filled with special liquid for the peritoneal cleansing, while the other bag is empty) under aseptic conditions, (as for example described in the EP-A-0 092 528) which are assured through an external infusion of antiseptic.

Such systems for exchanging liquids are already in existence combined with one (single systems) or two (double systems) bags. The main disadvantage of the existing systems is the high occurency frequency of peritonitis.

In order to reduce the occurency frequency of peritonitis an antiseptic is used in or out of the tubes. The use of an antiseptic in the tubes is made as follows:
a) By suction or infusion of the antiseptic from one end of the tubes of the bags. In this case, free tubes "Y" shaped are used. The vertical branch of the "Y" is connected to the peritoneal catheter and the antiseptic is infused or sucked through one (of the two free) lateral branches of the "Y". Then follows the connection of the bags of the peritoneal cleansing at the ends of the lateral branches of the "Y".A characteristic of the kinetics of the antiseptic is its movement from the end of one of its lateral branches, to the end of its other lateral branch, while the vertical branch of the "Y" is by-passed.
b) By the insertion of two spikes (usually metallic) in a plastic cartridge full of antiseptic. In this cas also, transfer tubes of liquids "Y" shaped or slightly modified "T" shaped are used. One of the spikes is at the end of the vertical branch of the "Y", while the other spike is fitted to the end of the peritoneal catheter or to the extension tube. The bags of the peritoneal cleansing are fitted to the ends of the lateral branches of the "Y". The two spikes enter the cartridge with the antiseptic just before the exchange of liquids. The whole procedure of the introduction and abduction of the solutions is carried out through the cartridge of the antiseptic, which is inserted between the two spikes.A characteristic of the kinetics of the antiseptic is the modification of its kinetic condition: while not moving (introduced beforehand in the cartridge) is carried away with turbulent flow to the empty bag. In other words, the use of the antiseptic is done through passive immersion of the spikes in a cup (cartridge) containing the antiseptic.

The advantage of this invention is the dynamic infusion (by syringe) of the antiseptic just before the exchange of liquids of the peritoneal cleansing, by means of a special floating tube (π1) installed in the tubes arrangement for liquid exchange. For the system F, the infusion of the antiseptic is assured on both sides of the conection point (ε) of the tubes (σ) of the bags with the extension tube (γ) of the peritoneal catheter.

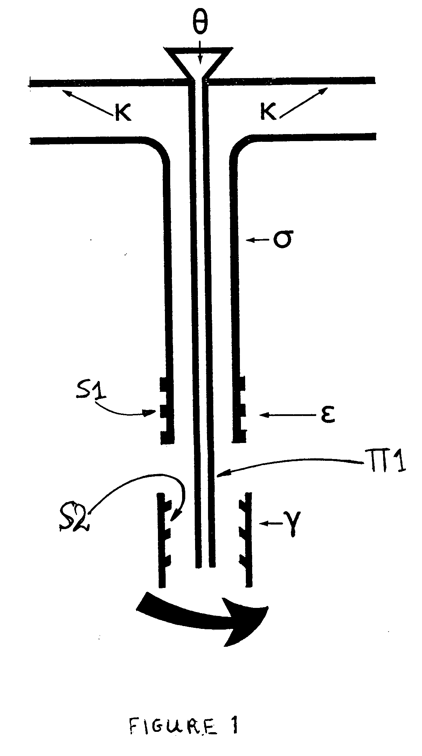

The system F is organized around the "T" shaped convergence point of the tubes of the bags. At both ends of the horizontal branch of the T (head-κ- of the T), the system bears the bags of the peritoneal cleansing (one full with liquid and one empty). The free end of the vertical branch (branch-σ- of the T), has a screw (S1), connected with a corresponding screw (S2), of the tube (γ) of the peritoneal catheter. In the middle of the T head, there is an infusion aperture (ϑ) for the antiseptic. The floating tube starts from the infusion aperture (ϑ) of the T head (Fig. 1), is borne within the T branch and throughout the course of the branch and projects from the connecting screw by 2-3 cm. Just before the exchange of liquids, the free end of the floating tube enters the tube extending the peritoneal catheter and the thread (S1) of the T is connected with the corresponding thread (S2) of the extension tube of the peritoneal catheter. Thus, just before the infusion of the antiseptic, the floating tube (π1) is located within the extension tube of the peritoneal catheter, while its free end lies between the closed clamp C1 (Fig. 1) of the extension tube of the peritoneal catheter and the point of connection "ε" (=connecting point of the system F, with the tube extending the peritoneal catheter).

After connecting the system F, with the extension tube of the peritoneal catheter, the infusion of the antiseptic is performed through the infusion aperture (ϑ) of the head of "T" by means of a common syringe. Due to the fact that the infusion aperture communicates hermetically only with the floating tube (π1), the antiseptic flows in the tubular float and sprays the area of the tubes, on both sides of the connecting point (ε). Thus, after this area is filled with antiseptic, the antiseptic flows backward (in the branch-σ-of the "T", but outside the tubular float -π-) to the empty bag. The tubes are washed out of the antiseptic during the evacuation of the peritoneal cavity to the empty bag of system F. Thereafter follows the filling of the peritoneal cavity with the liquid of the full bag of system F.

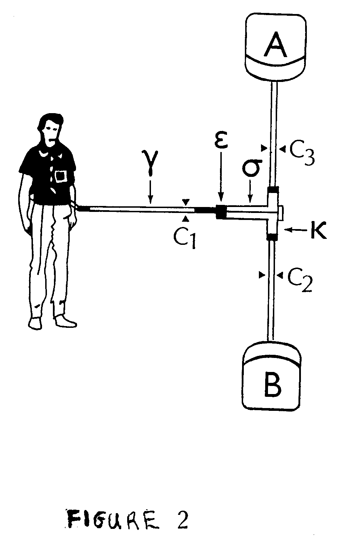

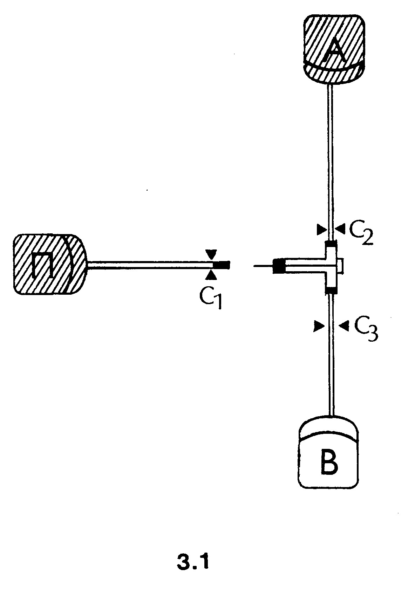

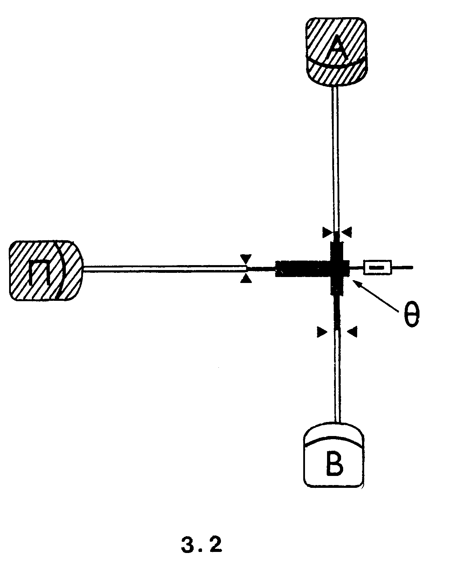

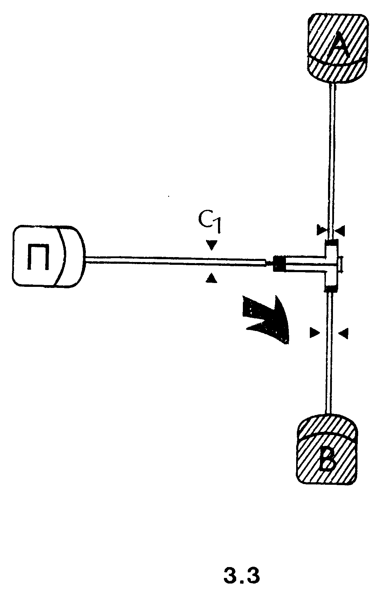

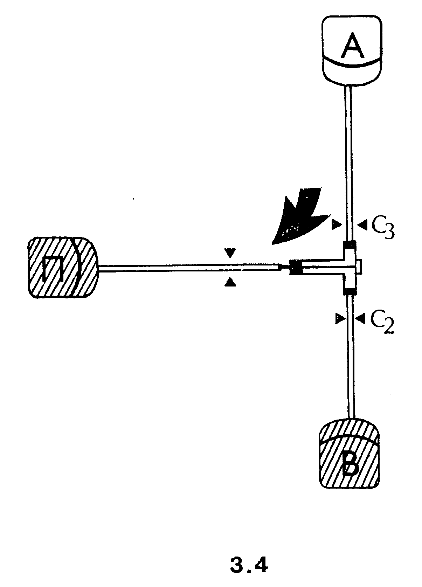

Figure 1 shows a section of point "T" together with the floating tube for the infusion of the antiseptic which projects from the connection screw by 2-3 cm. Figure 2 shows the connection of the system F with the extension tube (γ) of the peritoneal catheter of a patient. Figure 2 shows the location of the branch of "T" (indicated with "σ") as well as the head of the "T" (indicated with "κ") after connection of the system F with the tube extension of the peritoneal catheter of a patient. Figure 3 shows the successive exchange phases of liquids and the infusion of antiseptic during the operation of system F. The bag "π" in figure 3 represents the peritoneal cavity of a patient. Figure 3.1 shows the way the floating tube of system F extends from the branch of the "T", just before the connection of the system F with the extension tube of the peritoneal catheter. Figure 3.2 shows the infusion of antiseptic in system F from the infusion aperture of the "T", as well as the area of the initial distribution of the antiseptic between the clamps (C1, C2, C3) of the various tubes. Figure 3.3 shows the washing out of the tubes and of the "T" during the evacuation of the liquid which is in the bag "π". Figure 3.4 shows the filling of the bag "π" with the contents of the bag A.

A description is given below of one way of application of system F in relation to the drawings. System F comprises a point "T" (figure 1), which is the convergence point of two tubes starting from the two bags (A and B, figure 2): one bag full of liquid for peritoneal cleansing (A, figure 2) and one empty bag (B, figure 2). At the connection of the branch of the "T" (figures 1 and 2), with the extension tube (γ, figure 2) of the peritoneal catheter, the floating tube of "T" (figure 1) enters the lumen of the extension tube of the peritoneal catheter by about 2-3 cm (figure 2) protruding out of the connecting point "ε" (figure 2). Immediately after the above follow: 1) Infusion of the antiseptic through the aperture of infusion (ϑ) of the "T" (figure 3.2). Thus, the microbes which probably entered the system of tubes upon connection are exterminated. 2) Opening of the clamp (C1) of the tube of the bag "π", resulting in the supply of its contents to the bag "B" (figure 3.3) Thus, a wash out of system F is achieved by the antiseptic. 3) Closing of clamp (C2) of the tube of the bag "B" and opening the corresponding clamp (C3) of the tube of the bag "A" resulting in the flow of the contents of the bag "A" to the bag "π". In this way the bag "π" is filled with fresh solution (figure 3.4). After these manipulations, the system F is disconnected and disposed of.

## Claims

1. A device for the exchange of peritoneal dialysis comprising a T-shaped tube (or similar), each branch of which is to be connected to a peritoneal catheter (γ) and two bags (A, B), which device assures the asepsis of the tubes for the transfer of liquids, on both sides of the connection point of the peritoneal catheter to the device,
Characterised in that the infusion of the antiseptic is performed after having connected the device with the peritoneal catheter (γ) by means of a floating tube (or tubes) located within the main tube (σ) of the T-shaped device for the transfer of liquids of the peritoneal dialysis.

2. A device as claimed in claim (1), comprising a point of convergence of "T" shaped or variation of "T" (e.g. Y) tubes and which bears a floating tube (π1) (or tubes) for the infusion of antiseptic in a branch (or branches) of the point of convergence of the tubes of the bags.

3. The use of the device of claim 1 whereby the flow of the liquids therein has any direction.

4. A combination of a peritoneal catheter with a device as claimed in any of the claims 1 to 3.

5. A device as claimed in claim (4), which performs the external infusion of antiseptic, through an infusion aperture (or apertures), placed anywhere along the tubes for the exchange of liquids for the peritoneal dialysis and through the floating tube(s) placed in the wall of any the tube for the transfer of the liquids for the peritoneal dialysis.

## Patentansprüche

1. Ein Apparat, der zum Austausch von peritonealer Lösung dient, und der aus einem T-förmigen Rohr (oder ähnliches) besteht, an dessen Enden jeweils ein peritonealer Katheter (γ) und zwei Säcke (A,B) angeschlossen sind. Dieser Apparat sichert die Keimfreiheit der Rohre während der Übertragung der Flüssigkeit und zwar auf beiden Seiten der Verbindungsvorrichtung des peritonealen Katheters mit dem Apparat, gekennzeichnet dadurch, daß die Einführung des antiseptischen Mittels erst dann erfolgt, wenn der Apparat schon an den peritonealen Katheter (γ) über ein Rohr (oder Rohre) angeschlossen worden ist. Dieses Rohr (oder diese Rohre) schwimmt - bzw schwimmen - und befindet sich -bzw befinden sich - im Hauptrohr (σ) des Apparates (in T - Form), der zur Übertragung der peritonealen Lösung vorgesehen wird.

2. Ein Apparat nach Anspruch (1),der aus einem Treffpunkt der Rohrleitungen der Säcke, in T - Form bzw einer entsprechenden variierten Form (z.B.Y) besteht, und der einen rohrförmigen, zur Einspritzung von antiseptischem Mittel dienenden Schwimmer enthält - bzw mehr als einen Schwimmer -, der (bzw die)im einen Glied - bzw in den Gliedern-des Treffpunktes der Rohrleitungen der Säcke befindet - bzw befinden.

3. Dieser Anspruch betrifft den Gebrauch des Apparates, der den Anspruch (1) erfüllt. Bei der Anwendung dieses Apparates hat die Strömung der Flüssigkeiten im Apparat eine beliebige Richtung.

4. Eine Kombinierung eines peritonealen Katheters mit einem Apparat nach den Ansprüchen (1) und (3).

5. Ein Apparat nach Anspruch (4) mit dem die Einspritzung von antiseptischem Mittel von außen her gesichert wird. Die Einspritzung soll durch eine Öffnung - bzw mehr als eine Öffnungen - erfolgen,die irgendwo längst der Rohre zum Flüssigkeitenaustausch bei der peritonealen Entleerung liegt-bzw liegen, sowie auch durch Rohre (oder Schwimmer), die sich im Inneren der Rohre zur Übertragung der Flüssigkeiten aus der peritonealen Entleerung befinden.

## Revendications

1. Un appareil pour le changement de la solution péritonéale, qui comprend un tube en forme de T ( ou une forme similaire ) avec ses deux extrémités jointes à une sonde ( c ) et deux sacs ( A, B ) pour garantir l'asepsie des tubes pour le transport des liquides aux deux côtés du point de connexion de la sonde péritonéale avec l'appareil, qui est faite de façon que l'introduction de l'antiseptique puisse se faire après l'union de l'appareil avec la sonde péritonéale ( c ) à travers un tube ( ou tubes ) qui flotte(nt) et qui se trouve(nt) dans le tube principal ( s ) de l'appareil en forme de T pour le transport des liquides de la solution péritonéale .

2. Un appareil comme celui qui est décrit dans l'exigence No 1, constitué d'un point de convergence des conduits des sacs en forme de " T " ou d'une variante de T ( par exemple Y ) et qui porte un flotteur ( ou des flotteurs ) en forme de tube pour l'infusion de l'antiseptique ( p1 ) dans une branche ( ou branches ) du point de connexion des conduits des sacs .

3. L'utilisation de l'appareil de l'exigence No 1 à travers lequel le flux des liquides dans l'appareil peut prendre n'importe quelle direction .

4. Une combinaison de sonde péritonéale et d'appareil comme dans les exigences No 1 et 3 .

5. Un appareil comme celui décrit dans l'exigence No 4 qui garantit l'infusion d'antiseptique à travers une ouverture ( ou des ouvertures ) d'entrée, placé ( ou placés ) à n'importe quel point des conduits d'échange des liquides du nettoyage péritonéal et à travers les tubes ( ou les flotteurs ) placés au pelage ou au tuyau des conduits de transport des liquides du nettoyage péritonéal .
